# EUROPÄISCHE PATENTSCHRIFT

(11) **EP 2 801 335 B1**
(45) Veröffentlichungstag und Bekanntmachung des Hinweises auf die Patenterteilung: **14.03.2018**
(21) Anmeldenummer: 13166888.1
(22) Anmeldetag: 07.05.2013
(51) Int. Cl.: B05C 17/005, A61M 5/31, A61M 5/28, A61M 5/24, A61C 5/62, B65D 83/00

(54) **Spritze**
Syringe
Seringue

(43) Veröffentlichungstag der Anmeldung: 12.11.2014
(73) Patentinhaber: Ivoclar Vivadent AG, 9494 Schaan (LI)
(72) Erfinder: Müller, Frank, 6800 Feldkirch (AT)
(74) Vertreter: Baronetzky, Klaus

(56) Entgegenhaltungen:
- WO-A1-2007/122363
- DE-A1-102005 028 925

## Beschreibung

Die Erfindung betrifft eine Spritze, insbesondere für flüssige oder viskose Dentalmateriallien, gemäß dem Oberbegriff von Anspruch 1.

Eine derartige Spritze ist seit langem bekannt. Als Beispiel hierfür sei die bereits etwas neuere US 4,863,072 erwähnt. Bei dieser Spritze lässt sich ein Kolben zum Ausdrücken von Dentalmaterial in ein Spritzengehäuse einschrauben, um so eine gesteuerte und fein dosierbare Abgabe des Dentalmaterials zu ermöglichen.

Für eine raschere Abgabe des Dentalmaterials ist bei dieser Lösung auch eine Zwei-Hand-Bedienung möglich, bei welcher das Spritzengehäuse 12 mit einer Hand bzw. mit zwei Fingern dieser Hand gegriffen wird und der dortige Betätigungsknauf, der in Form einer Scheibe ausgebildet ist, rasch gedreht wird.

In diesem Zusammenhang wird es jedoch als unbefriedigend empfunden, dass der Zeitaufwand bei rascher Betätigung beträchtlich ist und ein rasches Betätigen durch eine schnelle manuelle Drehung erfordert, die als unangenehm empfunden wird.

Eine raschere Abgabe des dortigen zu injizierenden Materials ist praktisch nur durch schnelleres Verdrehen des Rendelrads möglich.

Zahlreiche weitere Spritzen 10 sind bereits vorgeschlagen worden. Als Beispiel sei die DE 199 00 792 C1 genannt, gemäß welcher eine Dosierung durch Verdrehen eines Rendelrads erfolgen soll. Diese Lösung ist recht kompliziert und erfordert auch passgenau hergestellte Einzelteile, was zu entsprechend hohen Herstellungskosten führt.

Das Dokument WO 2007/122363 A1 zeigt eine Spritze, welche ein Spritzengehäuse mit einem Kolben darin aufweist. Ferner weist die spritze eine Kanüle auf, welche an einem Ende des Spritzengehäuses angebracht ist, Das Spritzengehäuse hat einen verformbaren Bereich benachbart zur Kanüle.

Das Dokument DE 10 2005 028 925 A1 zeigt ein Handgerät zur Abgabe einer pastösen Füllmasse, insbesondere für dentale Zwecke, deren Viskosität durch Zuführung von Schwinungsenergie reduzierbar ist, mit einem Gerätegehäuse, mit einem Behälter für die pastöse Füllmasse, mit einer mit dem Behälter in Verbindung stehenden Austrittsdüse, mit einem Schwingungsgenerator und mit Druckerzeugungsmitteln zum Ausüben von Druck auf die pastöse Füllmasse.

Demgegenüber liegt der Erfindung die Aufgabe zugrunde, eine Spritze gemäß dem Oberbegriff von Anspruch 1 zu schaffen, die trotz der Verwendung eines kostengünstigen Herstellverfahrens eine rasche Spritzenentleerung, aber bei Bedarf auch eine feine Dosierung ermöglicht.

Diese Aufgabe wird erfindungsgemäß durch die Merkmale der Ansprüche 1 und 14 gelöst. Vorteilhafte Weiterbildungen ergeben sich aus den Unteransprüchen.

Erfindungsgemäß besonders günstig ist es, dass das Spritzengehäuse, in welchem der Kolben geführt ist, über einen Teil seiner Länge fest, also gegenüber dem Kolben unnachgiebig, ausgebildet ist, und über einen weiteren Teil seiner Länge verformbar ist, wobei bevorzugt auch dieser Teil des Spritzengehäuses vom Kolben durchdringbar ist. Durch die Kombination dieser Teile lässt sie nach Wahl des Benutzers das Dentalmaterial rasch ausdrücken, indem in an sich bekannter Weise auf den Kolben gedrückt und dieser translatorisch bewegt wird, oder aber zur Feindosierung der verformbare Teil zusammengedrückt wird, so dass Dentalmaterial ausgedrückt wird.

Es versteht sich, dass für das Wirksamwerden des Ausdrückens der Schubwiderstand des Kolbens, also die Haftreibung des Kolbens im Spritzengehäuse, größer als der Fließwiderstand des Dentalmaterials durch die Auslasskanüle hindurch ist. Dies ist bei flüssigen Dentalmaterialien ohnehin automatisch so, und zwar auch dann, wenn der Kolben sich bereits in dem verformbaren Teil des Spritzengehäuses befindet, also eine nahezu vollständige Entleerung bereits stattgefunden hat.

Auch bei viskosen Dentalmaterialien ist es günstig, wenn der Schiebewiderstand des Kolbens in dem Spritzengehäuse, also insofern die Haftreibung beim stillstehenden Kolben, an die Viskosität angepasst ist, also bei viskoserem Dentalmaterial eine größere Haftreibung eingestellt wird als bei flüssigerem Material.

Die Einstellung kann in beliebiger geeigneter Weise über die Wahl der Abmessungen des Kolbens einerseits und des Spritzengehäuses andererseits vorgenommen bzw. eingestellt werden. Bevorzugt weist das Spritzengehäuse einen Innendurchmesser auf, der etwas, beispielsweise um 0,2 mm, größer als der Außendurchmesser des zylindrischen Kolbens ist. In an sich bekannter Weise weist der Kolben an seinem vorderen, also der Auslasskanüle benachbarten, Ende eine Ringnut auf. In dieser ist in ebenfalls an sich bekannter Weise ein O-Ring aufgenommen. Der O-Ring überragt in radialer Richtung den Außendurchmesser des Kolbens beispielsweise um 0,4 mm. Hierdurch erfolgt die Abdichtung zwischen der Wand des Spritzengehäuses und dem Kolben, dessen O-Ring beim Einführen in das Spritzengehäuse zusammengedrückt wird und über den Verlauf der Schiebebewegung des Kolbens in gleicherweise zusammengedrückt verbleibt.

Beispielsweise kann die Ringnut eine Breite von 2 mm und eine radiale Tiefe von 1,6 mm aufweisen. Wenn eine O-Ring mit entsprechendem Durchmesser und einer Stärke von 2 mm eingesetzt wird, springt dieser dementsprechend um 2 mm - 1,6 mm = 0,4 mm nach Außen vor und wird beim Einführen in das Spritzengehäuse um 0,2 mm zusammengedrückt. Die Größe des Anpressdrucks entscheidet über den Schiebewiderstand des Kolbens, aber auch über die Haftreibung des Kolbens in dem Spritzengehäuse.

Um nun einen größeren Wert für die Haftreibung zu erzielen, kann entweder ein O-Ring mit einer Stärke von 2,1 mm eingesetzt werden, oder die Tiefe der Ringnut beispielsweise auf 1,5 mm verringert werden. In beiden Fällen lässt sich das erwünschte Ergebnis erzielen, dass beim Zusammendrücken des vorderen Teils des Spritzengehäuses Dentalmaterial aus der Auslasskanüle ausgedrückt wird, ohne dass das Ausdrücken dazu führen würde, dass sich der Kolben rückwärts bewegt.

Besonderes Augenmerk wird erfindungsgemäß auf die Führung des Kolbens im vorderen, also nachgiebigen Teil des Spritzengehäuses gelegt. Beispielsweise ist es möglich, den nachgiebigen Teil des Spritzengehäuses nach der Art eine Griffmulde lediglich an zwei aneinander gegenüberliegenden Bereichen des Spritzengehäuses zu realisieren, und das Spritzengehäuse im Übrigen sich formstabil bis zur Auslasskanüle erstrecken zu lassen.

Die Dimensionierung erfolgt jedenfalls so, dass ein "Überströmen", also ein Bewegen von Dentalmaterial über die Dichtung des Kolbens hinweg, sicher vermieden ist. Auch hier gilt, dass die relativen Abmessungen des Kolbens einerseits und des Spritzengehäuses andererseits an die Viskosität des Dentalmaterials anpassbar ist. Bei flüssigem Dentalmaterial müssen typischerweise keine besonderen Vorkehrungen getroffen werden, da das flüssige Dentalmaterial sich durch die Auslasskanüle leicht ausdrücken lässt, wenn die einander gegenüberliegenden Bereiche des verformbaren Teils des Spritzengehäuses zusammengedrückt werden.

Bei viskoserem Dentalmaterial kann es günstig sein, wenn die Elastizität des leicht verformbaren Bereichs des Spritzengehäuses ausgenutzt wird, um den Anpressdruck zu erhöhen. Hierzu kann das Spritzengehäuse dort - beispielsweise durch Wärmeschrumpfen - mit einem Untermaß gefertig sein, so dass die Dichtung dort dann beispielsweise nicht nur um 0,2 mm, sondern um 0,3 mm zusammengedrückt werden muss, wenn der Kolben in den verformbaren Bereich des Spritzengehäuses eindringen soll.

Erfindungsgemäß günstig ist es, wenn der verformbare Bereich des Spritzengehäuses der Auslasskanüle benachbart ist. Hierdurch wird zunächst erreicht, dass die Feindosierung auch dann zur Verfügung steht, wenn der Kolben sich über mehr als die Hälfte zum Auslassende hin bewegt hat, also die Spritze beispielsweise halb entleert ist. Der verformbare Bereich kann beispielsweise das vordere Viertel oder das vordere Fünftel des zylindrischen Teils des Spritzengehäuses abdecken. Das Spritzengehäuse lässt sich auch dann zusammendrücken, wenn der Kolben sich bereits etwas in den verformbaren Bereich hineinerstreckt hat, indem nämlich lediglich der der Auslasskanüle benachbarte Teil des verformbaren Bereichs dann zusammengedrückt wird.

Es versteht sich, dass gerade bei einem viskosen Dentalmaterial typischerweise kein Zurückfedern des verformbaren Bereichs nach Druckbetätigung erfolgt. Dies ist günstig, denn dadurch wird ein Ansaugen durch die Auslasskanüle verhindert, die ansonsten Verunreinigungen erzeugen würde. Vielmehr wird der verformbare Bereich erst beim weiteren Betätigen der Druckstange des Kolbens wieder auf Nennmaß gebracht, also auseinandergedrückt.

Insofern sind besondere Federeigenschaften des verformbaren Bereichs des Spritzengehäuses nicht erforderlich oder erwünscht, hingegen sehr wohl dessen begrenzte Verformbarkeit.

Die Verformbarkeit des vorderen Bereichs des Spritzengehäuses lässt sich in weiten Bereichen an die Erfordernisse anpassen und nach Belieben des Anwenders gestalten. So ist es gemäß einer Ausführungsform vorgesehen, ein aus einem harten Kunststoff bestehendes Spritzengehäuse als Zylinder zu fertigen, der vorne zwei Durchgriffsausnehmungen hat. In dieses Außengehäuse lässt sich dann ein Innengehäuse des Spritzengehäuses einschieben und an der gewünschten Stelle befestigen. Das Innengehäuse deckt dann die Aussparungen des Außengehäuses ab und an dieser Stelle ist das Spritzengehäuse dann nachgiebig und verformbar. Der Kolben kann das Innengehäuse dann über seine ganze zylindrische Länge durchtreten, und an dem Innengehäuse ist dann typischerweise die Auslasskanüle einstückig angeformt.

Diese Lösung hat den Vorteil, dass der Kolben bis zum vorderen Ende des zylinderförmigen Teils des Innengehäuses führbar ist, so dass keine oder ganz geringe Rückstände des vergleichsweise teuren Dentalmaterials in der Spritze bei deren Entleerung verbleiben.

Alternativ und mit den gleichen Vorteilen lässt sich das Außengehäuse ebenfalls bis ganz nach vorne führen, und durch Ausgestaltung von Zungen das Außengehäuse selbst verformbar realisieren. Durch Druck auf die Zungen des Außengehäuses wird dann das Innengehäuse zusammengedrückt und ermöglicht das feindosierte Abgeben von Dentalmaterialien.

Gemäß einer weiteren Alternative ist der Kolben in an sich bekannter Weise mit einer Drehspindel verbunden. Das Spritzengehäuse weist ein Innengewinde auf, und die Drehspindel führt den Kolben durch die Drehbewegung des Drehgriffs der Drehspindel zum Ausdrücken des Dentalmaterials. Diese Lösung erlaubt den Grunde nach bereits ein feineres Dosieren als Spritzen, bei denen ein Kolben rein translatorisch geführt ist.

Dennoch ist es erfindungsgemäß günstig, einen verformbaren Bereich des Spritzengehäuses vorzusehen, der sich nach vorne, also zur Auslasskanüle hin anschließt und an welchem durch leichtes Zusammendrücken der Wandung des Spritzengehäuses Dentalmaterial ausgedrückt wird. Dieses Feindosieren kann rasch und gezielt und auch mit visueller und sensorischer Kontrolle erfolgen.

Gemäß einer weiteren Ausführungsform ist der verformbare Bereich in dem Verlauf der Auslasskanüle selbst angeordnet. Bei dieser Ausgestalltung ist der verformbare Bereich nach der Art einer Pumpe ausgebildet, weist also zwei Rückschlagventile auf, eins vor und eins nach der Membran der Pumpe, so dass beim Zusammendrücken des dort angeordneten verformbaren Bereichs das Material nach vorner gedrückt wird, und zwar unabhängig von der Haftreibung des Kolbens im zylindrischen Teil des Spritzengehäuses.

In weiterer vorteilhafter Ausgestaltung ist es vorgesehen, den verformbaren Bereich oder mindestens einen Teil des verformbaren Bereich des Spritzengehäuses durchsichtig oder durchscheinend auszubilden. Durch diese an sich bekannte Maßnahme lässt sich leicht eine visuelle Kontrolle des Zustands der Spritze, aber auch des verbleibenden Dentalmaterials realisieren.

Weitere Vorteile, Einzelheiten und Merkmalen ergeben sich aus der nachfolgenden Beschreibung mehrerer Ausführungsbeispiele anhand der Zeichnungen.

Es zeigen:
- Fig. 1: eine leicht schematisierte Schnittansicht einer Ausführungsform einer erfindungsgemäßen Spritze;
- Fig. 2: ein Teil der Spritze gemäß Fig. 1, in einer seitlichen Draufsicht;
- Fig. 3: eine weitere Ausführungsform einer erfindungsgemäßen Spritze, unter Darstellung eines Teils dieser Spritze, nämlich eines Teils der Auslasskanüle;
- Fig. 4: eine schematische Ansicht einer weitere Ausführungsform einer erfindungsgemäßen Spritze;
- Fig. 5: eine schematische Ansicht einer weiteren Ausführungsform einer erfindunggemäßen Spritze; und

Fig. 6 Eine schematische Ansicht eines Ausführungsbeispiels, welches nicht Teil der beanspruchten Erfindung ist. Die in Fig. 1 dargestellte Spritze 10 weist ein Spritzengehäuse 12 auf, das in dem dargestellten Ausführungsbeispiel zweiteilig ausgebildet ist.

Das Spritzengehäuse 12 weist ein Außengehäuse 14 und ein Innengehäuse 16 auf. Das Außengehäuse 14 hat eine vergleichsweise große Wandstärke von beispielsweise 1 mm oder 2 mm. Es ist aus einem härter eingestellten Kunststoff ausgebildet.

Das Innengehäuse 16 weist eine demgegenüber wesentlich geringere Wandstärke von beispielsweise 0,2 bis 0,3 mm auf. Sowohl aufgrund der geringen Wandstärke als auch aufgrund der Materialwahl, nämlich eines etwas weicher eingestellten Kunststoffs, ist das Innengehäuse 16 an sich nachgiebig ausgebildet und insofern verformbar. Es ist jedoch in seinem überwiegenden Teil von dem Außengehäuse 14 abgestützt. Der Innendurchmesser des Außengehäuses entspricht dem Außendruchmesser des Innengehäuses 16 oder ist um wenige µ unterschiedlich.

Am vorderen Ende des Spritzengehäuses 12 ist eine Auslasskanüle 20 ausgebildet. Die Auslasskanüle 20 ist in an sich bekannter Weise konisch zulaufend ausgebildet, so dass die Strömungsquerschnittsfläche eingangsseitig der Auslasskanüle 20 beispielsweise das Zwanzigfache, bevorzugt etwa das Sechsfache des Strömungsquerschnitts am Austrittsende der Auslasskanüle 20 beträgt. Der Innendruchmesser des Einlassendes der Auslasskanüle 20 beträgt dementsprechend etwas das Zweieinhalbfache des Innendurchmesser am Auslassende der Auslasskanüle 20.

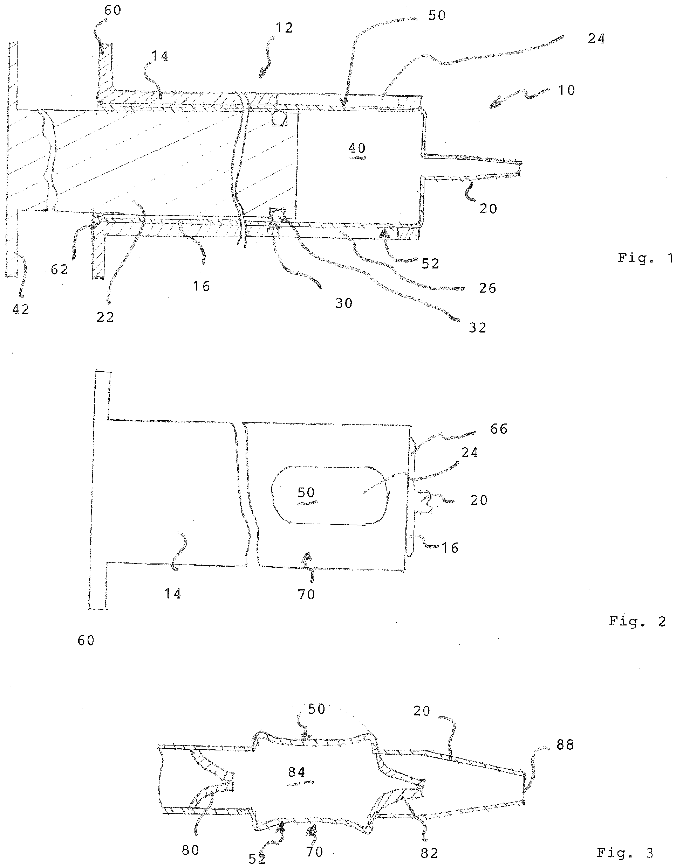

In ebenfalls an sich bekannter Weise ist die Auslasskanüle 20 in Strömungsverbindung mit dem zylindrischen Teil des Spritzengehäuses 12 verbunden. Der zylindrische Teil endet entweder in einem Konus mit einem sehr stumpfen Konuswinkel oder flach, wie es in Fig. 1 dargestellt ist, um so möglichst geringe Verluste an Dentalmaterial sicherzustellen. Bis zum Ende des zylindrischen Teils des Spritzengehäuses 12 ist ein Kolben 22 einschiebbar, um das vorher in der Spritze 10 aufgenommene Dentalmaterial auszudrücken.

In dem dargestellten Ausführungsbeispiel ist die Auslasskanüle 20 und das Zylinderende des zylindrischen Teils des Spritzengehäuses 12 einstückig mit dem Innengehäuse 16 ausgebildet. Es versteht sich, dass anstelle dessen aber auch eine beliebige andere geeignete Ausgestaltung wählbar ist.

Das Außengehäuse 14 weist zwei einander gegenüberliegende Durchbrechungen 24 und 26 auf. Mit diesen Durchbrechungen ist gewährleistet, dass die Außenseite des Innengehäuses 16 an dieser Stelle, die an der Außlasskanüle 20 benachbart ist, frei zugänglich ist.

Der Kolben 22 weist einen Durchmesser auf, der dem Innendurchmesser des Innengehäuses 16 im wesentlichen entspricht und um weniges, beispielsweise 0,1 mm, kleiner als dieser ist. An seinem vorderen, also dem der Auslasskanüle 20 zugewandten Ende ist eine Ringnut 30 ausgebildet, die einen Dichtungsring 32 aufnimmt. Der Außendurchmesser des Kolbens 22 mit eingelegtem Dichtungsring 32 ist etwas größer als der Innendurchmesser des Innengehäuses 16, beispeilsweise um 0,2 mm oder 0,3 mm. Der Dichtungsring 32 ist in an sich bekannter Weise elastisch kompressibel und erzeugt eine Abdichtung des Innenraums 40 der Spritze 10, der mit Dentalmaterial gefüllt ist.

Die Abdichtung durch den Dichtungsring 32 ist so stark, dass bei Druck auf den Kolben 22 über die Greifhandhabe 42 des Kolbens 22 Dentalmaterial aus dem Innenraum 40 über die Auslasskanüle 20 ausgedrückt wird, aber der Dichtungsring 32 gegen das Innengehäuse 16 abdichtet.

Auch bei Druck auf die freiliegenden Flächen 50 und 52 des Innengehäuses 16 unter den Durchbrechungen 24 und 26 strömt kein Dentalmaterial an dem Dichtungsring 32 vorbei. Beim Druck auf diese Flächen 50 und 52 wird der Kolben 22 auch nicht nach hinten gedrückt, da die Reibung zwischen dem Dichtungsring 32 und dem Innengehäuse 16 ausreichend groß ist, um dies zu verhindern. Vielmehr wird hierdurch feinfühlig Dentalmaterial aus der Auslasskanüle 20 ausgedrückt und abgegeben.

In dem dargestellten Ausführungsbeispiel weist das Außengehäuse 14 an seinem rückwärtigen Ende einen umlaufenden Kragen 60 auf. Für das Betätigen des Kolbens 22 nimmt der Benutzer die Spritze 10 zwischen Zeigefinger und Mittelfinger, so dass Zeigefinger und Mittelfinger von der vorderen Seite der Spritze 10 aus an dem Kragen 60 anliegen. Der Daumen der gleichen Hand des Benutzers liegt an der Greifhandhabe 42 an. Hierdurch lässt sich leicht in an sich bekannter Weise eine Einhandbedienung für die schnelle Abgabe von Dentalmaterial realisieren.

Bei Feindosierung wird der Griff seitens des Benutzers geändert. Die Spritze 10 wird nunmehr zwischen Daumen und Zeigefinger ausschließlich an den Flächen 50 und 52 gefasst, während der rückwärtige Teil der Spritze 10 einschließlich der Greifhandhabe 42 an dem Handballen anliegt und gegebenenfalls von dem Ringfinger stabilisiert wird. Beim Druck zwischen Zeigefinger und Daumen des Benutzers lässt sicht nun so feinfühlig Dentalmaterial abgeben, und der Benutzer ist hierbei bei der Abgabe des Dentalmaterials mit den gut steuerbaren Fingern Daumen und Zeigefinger ausgesprochen nah am Abgabeort, so dass sowohl die Dosierung als auch die positionelle Steuerung präzise und genau möglich ist.

Insofern lässt sich erfindungsgemäß trotz der Kombination der Abgabe mit größerer Menge als auch der Feindosierung eine Einhandbedienung realisieren, ohne dass die Herstellung der erfindungsgemäßen Spritze 10 besonders aufwändig wäre.

Für die Fixierung des Innengehäuses 16 an dem Außengehäuse 14 können diese an dem rückwärtigen Ende 62 miteinander verschweißt sein. Es ist aber auch möglich, dass dort das Innengehäuse 16 einen weiteren Kragen ausbildet, der ein Durchdrücken durch das Außengehäuse 14 verhindert.

Die Auslasskanüle 20 kann in an sich bekannter Weise außen und im rückwärtigen Bereich ein Außengewinde tragen, dass, für den Eingriff in ein Innengewinde einer Verschlusskappe bestimmt ist. Derartige Verschlusskappen und Systeme sind seit langem und an sich bekannt.

Aus Fig. 2 ist ersichtlich, in welcher Weise sich die Durchbrechung 24 greiffreundlich erstreckt und zugleich die Fläche 50 des Innengehäuses 16 freigibt. Wie ersichtlich ist, ragt das Innengehäuse 16 etwas nach vorne aus dem Außengehäuse vor, nachdem die radiale Abstützung dort durch die Stirnwand 66 des Innengehäuses 16 übernommen wird.

Die Dimensionierung der Materialstärken und Strömungswiderstände ist so bemessen, dass beim Ausdrücken des Dentalmaterials aus dem Innenraum 40 über die Betätigung des Kolbens 22 auch im Bereich der Durchbrechungen 24 und 26 kein Überströmen über den Dichtungsring 32 erfolgt. Hierzu sind die Durchbrechungen 24 und 26 ausreichend klein gewählt, so dass eine Abstützung auch im vorderen Bereich über das Außengehäuse 14 besteht. Alternativ kann auch das Außengehäuse selbst an dieser Stelle beweglich gehalten sein, beispielsweise über Schlitze, die ein Zusammendrücken partiell erlauben, oder auch über einseitige oder beidseitige Hebel, die außen an der Spritze 10 angebracht sind und dem Zusammendrücken dienen.

Eine weiter modifizierte Ausgestaltung der Erfindung ist aus Fig. 3 ersichtlich. Bei dieser Lösung ist der verformbare Teil des Spritzengehäuses 12 in die Auslasskanüle 20 verlagert. Bei der Ausführungsform gemäß Fig. 1 und Fig. 2 ist der verformbare Bereich 70 der Auslasskanüle 20 benachbart, während er bei der Ausführungsform gemäß Fig. 3 Teil dieser ist. Dementsprechend ist der gesamte zylindrische Teil des Spritzengehäuses 12 formstabil und ist durch die üblichen Kräfte nicht oder nicht wesentlich verformbar. Der verformbare Bereich 70 des Spritzengehäuses 12 weist bei dieser Lösung Flächen 50 und 52 auf, die aufeinander zu zusammendrückbar sind. Sie sind in dem dargestellten Ausführungsbeispiel fingerfreundlich gestaltet, können aber sowohl konkav als auch konvex ausgebildet sein. Stromab und stromauf dieses verformbaren Bereichs 50 sind je Rückschlagventile 80 und 82 ausgebildet.

Beim Zusammendrücken der Flächen 50 und 52 entsteht Druck im Innenraum 84 des verformbaren Bereichs 70. Da das Rückschlagventil 80 schließt, öffnet sich das Rückschlagventil 82, und Dentalmaterial kann über das Auslassende 88 der Auslasskanüle 20 abgegeben werden. Beim Loslassen der Flächen 20 und 22 möchte das Material der Auslasskanüle 20 wieder in den Ausgangszustand zurückkehren, nachdem es elastisch ist. Aufgrund der Wirkung des Rückschlagventils 82 kann das Dentalmaterial jedoch aber nicht zurückströmen, so dass sich das Rückschlagventil 80 öffnet und Material nachströmen kann.

Dieses wird über den Innenraum 40 nachgesaugt, oder durch Druck auf die Greifhandhabe 42 des Kolbens 22 nachgeschoben.

Es versteht sich, dass bei dieser Lösung gegebenenfalls auch auf das Rückschlagventil 82 verzichtet werden kann, wenn eine Rückströmen des Dentalmaterials aus dem Auslassende unkritisch sein sollte.

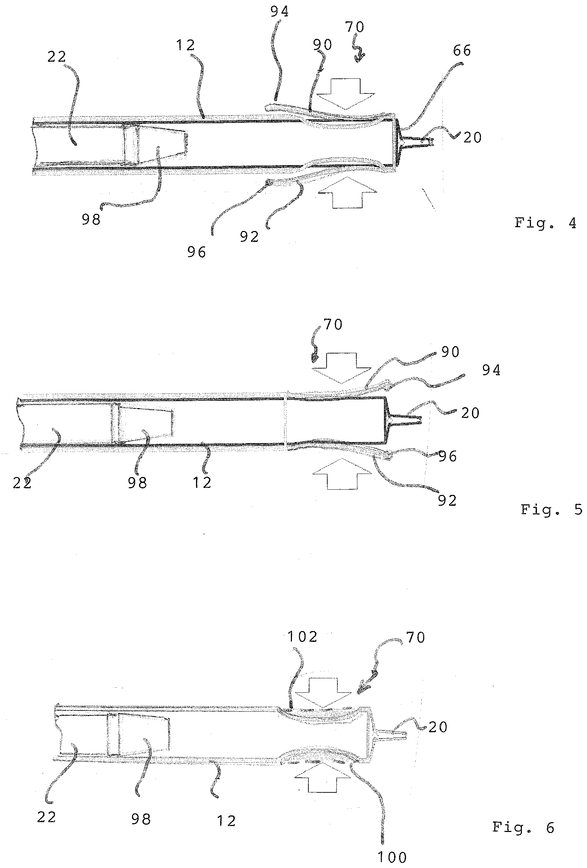

Die in Fig. 4 dargestellte Ausführungsform zeigt eine Spritze 10, bei der der Bereich 70 des Spritzengehäuses 12 verformbar ist. Die Verformung erfolgt über in dem der Auslasskanüle 20 benachbarten Bereich 70 vorgesehene elastische Seitenwände des Spritzengehäuses 12. Es sind Hebel 90 und 92 an dem vorderen Ende des zylindrischen Teils des Spritzengehäuses 12 der Stirnwand 66 benachbart angebracht. An dieser Stelle ist jeder Hebel 90 und 92 in beliebiger geeigneter Weise beweglich mit dem Spritzengehäuse 12 verbunden. In dem dargestellten Ausführungsbeispiel ist jeder Hebel im wesentlichen S-förmig geschwungen und, so dass er im Bereich 70 auf das Spritzengehäuse 12 drücken kann, wenn an den fernen Enden 94 und 96 je Druck auf den betreffenden Hebel 90 und 92 ausgeübt wird.

Mit dieser Ausgestaltung lässt sich noch feinfühliger die Abgabe von Dentalmaterial über die Auslasskanüle einstellen, denn der Hebel 90 - und gleichermaßen der Hebel 92 - wirkt untersetzend, so dass eine Bewegung der Enden 94 und 96 zu einer geringeren Bewegung im Bereich 70 führt.

In dem dargestellten Ausführungsbeispiel sind die Hebel 90 und 92 als einarmige Hebel ausgebildet, wobei der Lastarm je kürzer als der Kraftarm ist.

In dem dargestellten Ausführungsbeispiel ist ferner der Kolben 22 mit einem Konus 98 an seiner der Auslasskanüle 20 zugewandten Spitze versehen. Diese Lösung ermöglicht es, den Kolben 22 bis an die Stirnwand 66 anliegend nach vorne zu schieben, aber dennoch den verformbaren Bereich 70 zu verformen. Eine Feindosierung ist damit bis zu vollständigen Entleerung des Dentalmaterials möglich.

Eine ähnliche Ausgestaltung ist aus Fig. 5 ersichtlich. Auch hier sind Hebel 90 und 92 vorgesehen, über die untersetzend Druck auf den verformbaren Bereich 70 des Spritzengehäuses 12 ausübbar ist, wobei der Bereich 70 wiederum der Auslasskanüle 20 benachbart ist.

Auch hier weist der Kolben 22 an der Spitze einen Konus 98 auf, der störungsfrei in den verformbaren Bereich 70 eindringen kann.

Im Unterschied zu der Ausführungsform gemäß Fig. 4 ist bei der Ausführungsform gemäß Fig. 5 jeder Hebel 90 und 92 an dem der Auslasskanüle 20 gegenüber liegenden Ende des verformbaren Bereich 70 angelenkt. Dementsprechend weisen die Enden 94 und 96 nach vorne, also zur Auslasskanüle 20 hin. Auch hier lässt sich eine Untersetzung und eine entsprechend feinfühlige Dosierung der Materialabgabe erzielen. Beim Ausführungsbeispiel gemäß Fig. 6 ist es vorgesehen, den verformbaren Bereich 70 unmittelbar in das Spritzengehäuse einzuarbeiten. Das Spritzengehäuse ist bei diesem Ausführungsbeispiel dementsprechend nicht zweiwandig wie bei den Ausführungsformen gemäß Fig. 1 und Fig. 2 sondern einwandig. Der formstabile Bereich des Spritzengehäuses 12 weist im Grunde Durchbrechungen entsprechend den Durchbrechungen 24 und 26 aus Fig. 1 auf. Diese sind jedoch durch elastische Einsätze 100 und 102 geschlossen, so dass auch hier eine Feindosierung in der bereits beschriebenen Weise möglich ist.

## Patentansprüche

1. Spritze, insbesondere für viskose oder flüssige Dentalmaterialien, mit einem Spritzengehäuse (12), in welchem ein Kolben (22) in an sich bekannter Weise schiebebeweglich geführt ist, wobei der Kolben (22) insbesondere aus dem Spritzengehäuse (12) entnehmbar und in dieses einführbar ist, und mit einer Auslasskanüle (20), die an dem Spritzengehäuse (12) angebracht ist, wobei ein der Auslasskanüle (20) benachbarter Bereich des Spritzengehäuses (12) verformbar und ein von der Auslasskanüle (20) entfernter Bereich des Spritzengehäuses (12) formstabil ausgebildet ist, **dadurch gekennzeichnet, dass** der formstabile Bereich des Spritzengehäuses (12) durch einen aufgesetzten Spritzenkorpus (14) gebildet ist, der sich außen um ein Innengehäuse (16) erstreckt, das verformbar ausgebildet ist, wobei der Spritzenkorpus (14) zwei einander gegenüberliegende Durchbrechungen (24, 26) aufweist, sodass die Außenseite des Innengehäuses (16) an dieser Stelle, die der Auslasskanüle (20) benachbart ist, frei zugänglich ist.

2. Spritze nach Anspruch 1, **dadurch gekennzeichnet, dass** der verformbare Bereich des Spritzengehäuses(12) verformbare Seitenwände oder Seitenwandbereiche aufweist, deren Innenabmessungen den Innenabmessungen des formstabilen Bereichs des Spritzengehäuses (12) entsprechen.

3. Spritze nach Anspruch 2, **dadurch gekennzeichnet, dass** die Innenabmessungen des verformbaren Bereichs ein Einführen des Kolbens (22) in diesen erlauben und insbesondere minimal geringer als diejenigen des formstabilen Bereichs sind und dass der Reibungswiderstand zwischen dem Kolben (22) und dem verformbaren Bereich des Spritzengehäuses (12) um weniges, insbesondere zwischen 5 und 20%, höher als der Reibungs-widerstand zwischen dem Kolben (22) und dem formstabilen Bereich des Spritzengehäuses (12) ist.

4. Spritze nach einem der vorhergehenden Ansprüche, **dadurch gekennzeichnet, dass** die Auslasskanüle (20) an einem formstabilen Kanülenträger angebracht ist, der die Stirnfläche des Aufnahmevolumens der Spritze (10) - insofern dem Kolben (22) gegenüberliegend - abdeckt, zwischen welchem Kanülenträger und dem formstabilen Bereich des Spritzengehäuses (12) sich der verformbare Bereich des Spritzengehäuses (12) erstreckt.

5. Spritze nach einem der vorhergehenden Ansprüche, **dadurch gekennzeichnet, dass** der verformbare Bereich eine Länge zwischen 10 und 50%, bevorzugt zwischen 15 und 30% und insbesondere zwischen 20 und 25% der Länge des Spritzengehäuses (12) einnimmt, und/oder dass der verformbare Bereich des Spritzengehäuses (12) eine Länge zwischen 1 cm und 3cm, insbesondere etwa 2cm hat.

6. Spritze nach einem der vorhergehenden Ansprüche, **dadurch gekennzeichnet, dass** der verformbare Bereich durch eine zylindrische Tube gebildet ist, die in den
formstabilen Spritzenkorpus eingesetzt ist und mit diesem durch Verschweißen, Verkleben oder in beliebiger anderer geeigneter Weise unverlierbar verbunden ist.

7. Spritze nach einem der vorhergehenden Ansprüche, **dadurch gekennzeichnet, dass** der verformbare Bereich, der der Auslasskanüle (20) benachbart ist, nach der Art einer Tube zusammenquetschbar und entleerbar ist.

8. Spritze nach einem der vorhergehenden Ansprüche, **dadurch gekennzeichnet, dass** die Auslasskanüle (20) ein Rückschlagventil aufweist, das das Einsaugen von Luft bei Entlastung des Kolbens (22) und/oder des verformbaren Bereichs verhindert.

9. Spritze nach einem der vorhergehenden Ansprüche, **dadurch gekennzeichnet, dass** der formstabile Bereich des Spritzengehäuses (12) über den verformbaren Bereich des Spritzengehäuses (12) eine Verlängerung aufweist, die insbesondere bis zu einem Träger der Auslasskanüle (20) reicht und Betätigungsbereiche belässt, an welchen der verformbare Bereich des Spritzengehäuses (12) frei liegt.

10. Spritze nach einem der vorhergehenden Ansprüche, **dadurch gekennzeichnet, dass** die Spritze (10) Hebel aufweist, die sich über den verformbaren Bereich erstrecken und insbesondere an dem formstabilen Bereich angelenkt sind und über welche Druck auf den verformbaren Bereich des Spritzengehäuses (12) ausübbar ist.

11. Spritze nach Anspruch 10, **dadurch gekennzeichnet, dass** die Hebel als einseitige Hebel ausgebildet sind und ihre freien Enden sich im Wesentlichen parallel zur Längsachse der Spritze (10) erstrecken und zur Auslasskanüle (20) oder bevorzugt nach hinten weisen.

12. Spritze nach einem der Ansprüche 10 oder 11, **dadurch gekennzeichnet, dass** ein Hebel oder mehrere Hebel vorgesehen sind, deren Übersetzungen insbesondere unterschiedlich sind.

13. Spritze nach einem der vorhergehenden Ansprüche, **dadurch gekennzeichnet, dass** der verformbare Bereich zusammen mit der Auslasskanüle (20) durch ein in sich einstückiges und insbesondere quetschbares oder stauchbares Kunststoffteil gebildet ist, das in den formstabilen Teil der Spritze (10) eingesetzt ist, wobei insbesondere dessen Innendurchmesser geringer als der Innendurchmesser des formstabilen Teils des Spritzengehäuses (12) ist.

14. Spritze für viskose oder flüssige Dentalmaterialien, mit einem Spritzengehäuse (12), in welchem ein Kolben (22) in an sich bekannter Weise schiebebeweglich geführt ist, wobei der Kolben (22) insbesondere aus dem Spritzengehäuse (12) entnehmbar und in dieses einführbar ist, und mit einer Auslasskanüle (20), die an dem Spritzengehäuse (12) angebracht ist, wobei ein von der Auslasskanüle (20) entfernter Bereich des Spritzengehäuses (12) formstabil ausgebildet ist und den Kolben (22) schiebebeweglich führt,
**dadurch gekennzeichnet, dass** ein in der Auslasskanüle (20) angeordneter Bereich verformbar ist, nach der Art einer Pumpe ausgebildet ist, und zwei Rückschlagventile (80, 82) aufweist, wobei ein Rückschlagventil (80) vor und ein Rückschlagventil (82) nach der Membran der Pumpe angeordnet ist, sodass beim Zusammendrücken des dort angeordneten verformbaren Bereichs (70) das Dentalmaterial nach vorne gedrückt wird.

## Claims

1. A syringe, especially for viscous or,liquid dental materials, with a syringe housing (12), In which a piston (22) is slidably guided in a manner known per se, wherein the piston (22) especially is removable from the syringe housing (12) and is insertable thereinto, and with an outlet cannula (20), which Is attached to the syringe housing (12), wherein a region of the syringe housing (12) adjacent to the outlet cannula (20) is deformably formed and a region of the syringe housing (12) spaced apart from the outlet cannula (20) is formed In a dimensionally stable manner, **characterized in that** the dimensionally stable region of the syringe housing (12) is formed by a syringe body (14) attached on top, which extends on the outside around an inner housing (16), which is deformably formed, wherein the syringe body (14) comprises two holes (24, 26) opposite to each other, so that the outside of the inner housing (16) is freely accessible at that position adjacent to the outlet cannula (20).

2. The syringe according to Claim 1, **characterized in that** the deformable region of the syringe housing (12) comprises deformable side walls or side wall regions, the Inner dimensions of which correspond to the inner dimensions of the dimensionally stable region of the syringe housing (12).

3. The syringe according to Claim 2, **characterized In that** the Inner dimensions of the deformable region allow insertion of the piston (22) therinto and especially are minimally lower than those of the dimensionally stable region and **in that** the frictional resistance between the piston (22) and the deformable region of the syringe housing (12) is slightly larger, especially between 5 and 20%, than the frictional resistance between the piston (22) and the dimensionally stable region of the syringe housing (12).

4. The syringe according to one of the preceding claims, **characterized In that** the outlet cannula (20) is attached to a dimensionally stable cannula support, which covers the frontal face of the capacity of the syringe (10) - opposite to the piston (22), respectively - between which cannula support and the dimensionally stable region of the syringe housing (12) the deformable region of the syringe housing (12) extends.

5. The syringe according to one of the preceding claims, **characterized In that** the deformable region occupies a length between 10 and 50%, preferably between 15 and 30% and especially between 20 and 25% of the length of the syringe housing (12), und/or in that the deformable region of the syringe housing (12) has a length between 1 cm and 3cm, especially about 2cm.

6. The syringe according to one of the preceding claims, **characterized in that** the deformable region is formed by a cylindrical tube that is inserted into the dimensionally stable syringe body and is connected thereto by welding, bonding, or is undetachably connected thereto in any other suitable manner.

7. The syringe according to one of the preceding claims, **characterized in that** the deformable region, which is adjacent to the outlet cannula (20), is squeezable and dischargeable in a tube like manner.

8. The syringe according to one of the preceding claims, **characterized in that** the outlet cannula (20) comprises a check valve, which prevents intake of air during relief of load on the piston (22) und/or of the deformable region.

9. The syringe according to one of the preceding claims, **characterized in that** the dimensionally stable region of the syringe housing (12) comprises an extension across the deformable region of the syringe housing (12), which especially extends to a support of the outlet cannula (20), and leaving actuation ranges, where the deformable region of the syringe housing (12) is exposed.

10. The syringe according to one of the preceding claims, **characterized in that** the syringe (10) comprises levers extending across the deformable region and especially are articulated to the dimensionally stable region and via which levers pressure is applicable to the deformable region of the syringe housing (12).

11. The syringe according to Claim 10, **characterized in that** the levers are formed as unilateral levers, their free ends essentially extending parallel to the longitudinal axis of the syringe (10) and facing the outlet cannula (20) or preferably facing rearwards.

12. The syringe according to one of the Claims 10 or 11, **characterized in that** a lever or several levers are provided, the transmissions of which especially are different.

13. The syringe according to one of the preceding claims, **characterized in that** the deformable region, together with the outlet cannula (20), is formed by an integral and especially squeezable or compressible plastic member, which is inserted into the dimensionally stable portion of the syringe (10), wherein especially the inner diameter thereof is lower than the inner diameter of the dimensionally stable portion of the syringe housing (12).

14. The syringe for viscous or liquid dental materials, with a syringe housing (12), in which a piston (22) is slidably guided in a manner known per se, wherein the piston (22) especially is removable from the syringe housing (12) and is insertable thereinto, and with an outlet cannula (20), which is attached to the syringe housing (12), wherein a region of the syringe housing (12) spaced apart from the outlet cannula (20) is formed in a dimensionally stable manner and slidably guiding the piston (22), **characterized in that** a region arranged in the outlet cannula (20) is deformable, and is formed in a pump type manner, and comprises two check valves (80, 82), wherein one check valve (80) is arranged In front of the membrane of the pump and one check valve (82) is arranged behind the membrane, so that during compressing the deformable region (70) located thereat, the dental material is pressed forwards.

## Revendications

1. Seringue, en particulier pour les matériaux dentaires visqueux ou liquides, avec un cylindre de seringue (12) dans lequel un piston (22) est guidé de façon connue en soi de manière à pouvoir être déplacé par mouvement glissant, où, en particulier, le piston (22) peut être retiré du cylindre de la seringue (12) et peut être inséré dans celui-ci, et avec une canule de sortie (20), qui est attaché au cylindre de la seringue (12), où une zone du cylindre de la seringue (12) contiguë à la canule de sortie (20) est formée de manière déformable et une zone du cylindre de la seringue (12) éloignée de la canule de sortie (20) est formée de manière non-déformable, **caractérisée en ce que** la partie non-déformable du cylindre de la seringue (12) est formée par un corps d'injection posée (14), qui s'étend à l'extérieur autour d'un boîtier interne (16) qui est configuré déformable, où le corps de seringue (14) présente deux perforations (24, 26) disposées à l'opposé l'une de l'autre, de manière que la face externe du boîtier interne (16) est librement accessible à cet endroit contigu à la canule de sortie (20).

2. Seringue selon la revendication 1, **caractérisée en ce que** la zone déformable du boîtier de la seringue (12) présente des parois latérales ou des zones de parois latérales déformables, dont les dimensions intérieures correspondent aux dimensions intérieures de la zone non-déformable du cylindre de la seringue (12).

3. Seringue selon la revendication 2, **caractérisé en ce que** les dimensions intérieures de la zone déformable permettent d'insérer le piston (22) dans celle-ci et en particulier sont à peine inférieures à celles de la zone non-déformable et que la résistance au frottement entre le piston (22) et la zone déformable du cylindre de la seringue (12) est légèrement, en particulier entre 5 et 20%, supérieure à la résistance au frottement entre le piston (22) et la zone non-déformable du cylindre de la seringue (12).

4. Seringue selon l'une des revendications précédentes, **caractérisée en ce que** la canule de sortie (20) est montée sur un support de canule non-déformable, qui couvre - dans la mesure où il est en face du piston (22) - la face avant du volume de réception de la seringue (10), entre ledit support de canule et la zone non-déformable du cylindre de la seringue (12) s'étend la zone déformable du cylindre de la seringue (12).

5. Seringue selon l'une des revendications précédentes, **caractérisée en ce que** la zone déformable occupe une longueur entre 10 et 50%, de préférence entre. 15 et 30% et en particulier entre 20 et 25% de la longueur du cylindre de la seringue (12), et/ou que la zone déformable du cylindre de la seringue (12) a une longueur comprise entre 1 et 3 cm, en particulier environ 2 cm.

6. Seringue selon l'une des revendications précédentes, **caractérisée en ce que** la zone déformable est formée par un tube cylindrique, qui est inséré dans le corps non-déformable de la seringue et est raccordée avec celle-ci de façon imperdable par soudure, collage ou toute autre manière appropriée.

7. Seringue selon l'une des revendications précédentes, **caractérisée en ce que** la zone déformable, qui jouxte celle de la canule de sortie (20), est comprimable et vidangeable à la manière d'un tube.

8. Seringue selon l'une des revendications précédentes, **caractérisée en ce que** le tuyau de sortie (20) possède un clapet anti-retour, qui empêche l'aspiration d'air lors de la décharge du piston (22) et/ou de la zone déformable.

9. Seringue selon l'une des revendications précédentes, **caractérisée en ce que** la partie non-déformable du cylindre de la seringue (12) présente un prolongement par la partie déformable du cylindre de la seringue (12), qui atteint en particulier le support de la canule de sortie (20) et laisse des zones d'activation auxquelles la zone déformable du cylindre de la seringue (12) est exposée.

10. Seringue selon l'une des revendications précédentes, **caractérisée en ce que** la seringue (10) possède des leviers qui s'étendent le long de la zone déformable et en particulier sont articulés à la zone de forme non-déformable et par lesquels de la pression est exercée sur la zone déformable du cylindre de la seringue (12).

11. Seringue selon la revendication 10, **caractérisée en ce que** les leviers sont configurés comme leviers unilatérales et leurs extrémités libres s'étendent essentiellement parallèles à l'axe longitudinal de la seringue (10) et de ia canule de sortie (20) ou de préférence Inclinés à l'arrière.

12. Seringue selon l'une des revendications 10 ou 11, **caractérisée en ce qu'**un ou plusieurs leviers sont prévues, dont notamment les rapports de transmission sont différents.

13. Seringue selon l'une des revendications précédentes, **caractérisée en ce que** la zone déformable ainsi que la canule de sortie (20) sont formées en une seul pièce en plastique, qui en particulier peut être comprimée ou aplatie, qui est insérée dans la zone non-déformable de la seringue (10), où en particulier son diamètre intérieur est inférieur au diamètre intérieur de la zone non-déformable du cylindre de la seringue (12).

14. Seringue pour matériaux dentaires visqueux ou liquides avec un cylindre de seringue (12) dans lequel un piston (22) est guidé de façon connue en soi de manière à pouvoir être déplacé par mouvement glissant, où une zone du cylindre de la seringue (12) éloignée de la canule de sortie (20) est formée de manière non-déformable et guide le piston (22) par mouvement glissant, **caractérisée en ce qu'**une zone de la canule de sortie (20) est déformable, est formée à la manière d'une pompe et présente deux un clapets anti-retour (80, 82) ou un clapet anti-retour est disposé devant et un clapet anti-retour (82) est disposé derrière le diaphragme de la pompe, afin que lors de la compression de la zone déformable (70) s'y trouvant le matériau dentaire est poussé vers l'avant.
